# EUROPÄISCHE PATENTANMELDUNG

(11) **EP 3 747 482 A1**
(43) Veröffentlichungstag der Anmeldung: **09.12.2020**
(21) Anmeldenummer: 20181017.3
(22) Anmeldetag: 23.11.2010
(51) Int. Cl.: A61M 1/36

(54) **VERFAHREN ZUM VORUEBERGEHENDEN UNTERBRECHEN EINER EXTRAKORPORALEN BLUTBEHANDLUNG, STEUERVORRICHTUNG UND BLUTBEHANDLUNGSVORRICHTUNG**

(30) Priorität: 24.11.2009 DE 102009054415; 10.03.2010 DE 102010010928
(62) Teilanmeldung aus: 10784700.6
(71) Anmelder: Fresenius Medical Care Deutschland GmbH, 61352 Bad Homburg (DE)
(72) Erfinder: Herrmann, Uwe, 14558 Nuthetal (DE); Kipp, Sabine, 61348 Bad Homburg (DE); Nachbaur-Sturm, Christine, 53783 Eitorf (DE); Pusinelli, Thomas, 63674 Altenstadt (DE); Gruendken, Martin, 61191 Rosbach (DE); Verch, Georg, 65191 Wiesbaden (DE)
(74) Vertreter: Bobbert & Partner Patentanwälte PartmbB

(57) **Zusammenfassung**

Die vorliegende Erfindung betrifft ein Verfahren zum vorübergehenden Unterbrechen einer extrakorporalen Behandlung von Blut eines Patienten (200) mittels einer Blutbehandlungsvorrichtung (1). Das Verfahren umfasst das Ansteuern einer Steuer- oder Regeleinrichtung (3), welche dazu vorgesehen und konfiguriert ist, um die Blutbehandlungsvorrichtung (1) in einen Zustand zu versetzen, in welchem die Blutbehandlungssitzung des Patienten (200) zu deren Wiederaufnahme vorübergehend unterbrochen werden kann. Die vorliegende Erfindung betrifft ferner eine Steuer- oder Regeleinrichtung (3), eine Blutbehandlungsvorrichtung (1), ein digitales Speichermedium, ein Computer-Programm-Produkt sowie ein Computer-Programm.

## Beschreibung

Die vorliegende Erfindung betrifft ein Verfahren zum vorübergehenden Unterbrechen einer extrakorporalen Blutbehandlung gemäß Anspruch 1. Sie betrifft ferner eine Steuer- oder Regeleinrichtung gemäß Anspruch 9, eine Blutbehandlungsvorrichtung gemäß Anspruch 12, ein digitales Speichermedium gemäß Anspruch 14, ein Computer-Programm-Produkt gemäß Anspruch 15 sowie ein Computer-Programm gemäß Anspruch 16.

Aus verschiedenen Gründen können vorübergehende Unterbrechungen einer extrakorporalen Blutbehandlung erforderlich werden. Zu diesen Gründen zählen das Legen eines neuen Zugangs, das Durchführen von medizinischen Untersuchungen, ein Toilettenbesuch des Patienten, usw. Ihnen ist gemeinsam, dass nach Wegfall des Grundes für die vorübergehende Unterbrechung mit der extrakorporalen Blutbehandlung fortgefahren werden soll.

Eine Aufgabe der vorliegenden Erfindung ist, ein weiteres Verfahren zum vorübergehenden Unterbrechen einer extrakorporalen Blutbehandlung vorzuschlagen. Ferner sollen geeignete Vorrichtungen angegeben werden.

Diese Aufgabe wird durch ein Verfahren mit den Merkmalen des Anspruchs 1 gelöst.

So wird erfindungsgemäß ein Verfahren zum vorübergehenden Unterbrechen einer extrakorporalen Blutbehandlung eines Patienten bzw. einer extrakorporalen Behandlung von Blut des Patienten mittels einer Blutbehandlungsvorrichtung, mit welcher der Patient zum Zwecke der Blutbehandlung konnektiert ist, vorgeschlagen. Das Verfahren umfasst das Ansteuern einer Steuer- oder Regeleinrichtung, welche dazu vorgesehen und konfiguriert ist, um die Blutbehandlungsvorrichtung in einen Zustand zu versetzen, in welchem die Blutbehandlungssitzung durch Dekonnektieren des Patienten von der Blutbehandlungsvorrichtung zu deren Wiederaufnahme vorübergehend unterbrochen werden kann.

Vorteilhafte Weiterentwicklungen des erfindungsgemäßen Verfahrens sind jeweils Gegenstand von Unteransprüchen.

Der Begriff "vorübergehendes Unterbrechen", wie er hierin verwendet wird, bezeichnet ein zeitlich begrenztes Unterbrechen des Verfahrens. Der Zeitraum bzw. die Zeitdauer der Unterbrechung kann beliebig bzw. variabel vorgegeben bzw. eingestellt werden. Eine durchschnittliche Unterbrechungsdauer kann zwischen 0,5 - 4h betragen. Aber auch wenige Minuten oder mehr als 4h können unter "vorübergehend" fallen.

Die vorübergehende Unterbrechung kann mit dem Ziel der Wiederaufnahme der unterbrochenen Sitzung der extrakorporalen Blutbehandlung erfolgen. Sie kann mit dem Ziel erfolgen, den Patienten vorübergehend zu dekonnektieren und/oder diesen oder die Blutbehandlungsvorrichtung in einen dekonnektierbaren Zustand zu bringen. Das Dekonnektieren des Patienten kann, muss aber nicht Teil des Verfahrens sein.

"Vorübergehend" kann daher erfindungsgemäß dahingehend verstanden werden, dass im Moment des Unterbrechens die Absicht besteht, die Behandlung später wieder aufzunehmen - gleich, ob es später dazu kommt, oder nicht.

Die vorübergehende Unterbrechung erfolgt in bestimmten erfindungsgemäßen Ausführungsformen mit dem Ziel der Wiederaufnahme der unterbrochenen Sitzung der extrakorporalen Blutbehandlung mittels wenigstens einer der (oder mehrerer oder aller) bereits vor der Unterbrechung zur Blutbehandlung verwendeten medizintechnischen Funktionseinrichtungen. Damit kann ein vorzeitiges Verwerfen der verwendeten (einen, mehreren oder allen) medizintechnischen Funktionseinrichtung(en) oder Teilen hiervon vorteilhaft entfallen. Ferner kann ein zeit- und müheintensives (vollständiges oder teilweises) Ab- und ein erneutes Aufrüsten der Blutbehandlungsvorrichtung mit neuen bzw. unbenutzten oder sterilen medizintechnischen Funktionseinrichtungen vorteilhaft entfallen. In einigen dieser Ausführungsformen ist die Wiederaufnahme mit der bereits vor der Unterbrechung hierzu verwendeten medizintechnischen Funktionseinrichtung bereits im Moment des Unterbrechens geplant oder beabsichtigt.

Zu medizintechnischen Funktionseinrichtungen zählen in manchen erfindungsgemäßen Ausführungsformen Blutschläuche, Blutschlauchsätze, Blutkassetten, Trays, Organizer und dergleichen. Sie sind optional als Wegwerfartikel oder als Einwegartikel oder Disposables ausgestaltet oder eingesetzt. Der Begriff "extrakorporale Blutbehandlung", wie er hierin verwendet wird, bezeichnet eine außerhalb des Körpers eines Patienten stattfindende bzw. erfolgende Blutbehandlung. Eine derartige Blutbehandlung kann beispielsweise der Reinigung und/oder dem Austausch des Bluts eines Patienten dienen.

Die extrakorporale Blutbehandlung kann mittels eines extrakorporalen Blutkreislaufs erfolgen. Der extrakorporale Blutkreislauf kann ein Schlauchsystem bzw. ein Patientenschlauchsystem aufweisen. Das Patientenschlauchsystem kann eine Blutentnahmeleitung und eine Blutrückgabeleitung aufweisen.

Der Begriff "Blutentnahmeleitung", wie er hierin verwendet wird, bezeichnet eine Leitung oder einen Leitungsabschnitt des extrakorporalen Blutkreislaufs, welcher dazu vorgesehen ist, Blut vom Patienten bzw. aus dem Blutkreislauf des Patienten wegzuleiten bzw. abzuführen.

Der Begriff "Blutrückgabeleitung", wie er hierin verwendet wird, bezeichnet eine Leitung oder einen Leitungsabschnitt des extrakorporalen Blutkreislaufs, welcher dazu vorgesehen ist, Blut zum Patienten bzw. zum Blutkreislauf des Patienten hinzuleiten bzw. in diesen zurückzuführen.

Die Blutentnahmeleitung kann zum Beispiel eine arterielle Leitung oder ein arterieller Leitungsabschnitt des extrakorporalen Blutkreislaufs sein. Der Begriff "arterielle Leitung oder arterieller Leitungsabschnitt" bezeichnet dabei einen Teil des extrakorporalen Blutkreislaufs, welcher mit einer Arterie des Blutkreislaufs des Patienten verbunden ist.

Wenn die Blutentnahmeleitung ein arterieller Leitungsabschnitt des extrakorporalen Blutkreislaufs ist, kann die Blutrückgabeleitung entsprechend eine venöse Leitung bzw. ein venöser Leitungsabschnitt des extrakorporalen Blutkreislaufs sein. Der Begriff "venöse Leitung bzw. venöser Leitungsabschnitt" bezeichnet entsprechend einen Teil des extrakorporalen Blutkreislaufs, welcher mit einer Vene des Blutkreislaufs des Patienten verbunden ist.

In anderen erfindungsgemäßen Fällen kann die Blutentnahmeleitung ein venöser Leitungsabschnitt des extrakorporalen Blutkreislaufs sein, und die Blutrückgabeleitung kann entsprechend ein arterieller Leitungsabschnitt sein. Letzteres ist u.a. bei der Blutbegasung mit Sauerstoff regelmäßig üblich.

Das Patientenschlauchsystem kann Patientenschlauchklemmen aufweisen, welche dazu vorgesehen sind, nach ihrem Schließen bzw. durch ihr Schließen einen Blutfluss oder Blutstrom innerhalb eines Leitungsinneren der Blutentnahmeleitung und/oder der Blutrückgabeleitung zu unterbrechen bzw. zu stoppen. Entsprechend kann nach bzw. durch Öffnen der entsprechenden Patientenschlauchklemme vorgesehen sein, den Blutfluss oder Blutstrom innerhalb des Leitungsinneren der Blutentnahmeleitung und/oder der Blutrückgabeleitung fortzusetzen.

Es können wenigstens zwei Patientenschlauchklemmen im extrakorporalen Blutkreislauf vorgesehen sein; beispielsweise kann wenigstens eine Patientenschlauchklemme in bzw. an der Blutentnahmeleitung vorgesehen sein, und wenigstens eine weitere Patientenschlauchklemme kann in bzw. an der Blutrückgabeleitung vorgesehen sein.

Der Begriff "Patient", wie er hierin verwendet wird, bezeichnet ein Lebewesen, wie einen Menschen oder ein Tier. Bei einem Patienten kann es sich um einen Gesunden oder einen Kranken handeln.

Eine "Blutbehandlungsvorrichtung" schließt Vorrichtungen zum Durchführen einer Blutbehandlung wie einer Blutreinigung, z. B. einer Dialyse, einer Hämofiltration, einer Hämodiafiltration, einer Apherese, einer Oxygenierung (z. B. eine Blutbegasung mit Sauerstoff), einer Adsorption oder dergleichen, ein.

Der Begriff "Steuer- oder Regeleinrichtung", wie er hierin verwendet wird, bezeichnet eine Einrichtung, welche dazu ausgelegt, konfiguriert und/oder vorgesehen ist, um mit der Blutbehandlungsvorrichtung in funktionelle Wirkung zu treten, um - ggf. neben anderen Wirkungen - eine Unterbrechung der extrakorporalen Blutbehandlung zu veranlassen.

Die Steuer- oder Regeleinrichtung kann ausgestaltet sein, eine Automatisierung bzw. einen automatischen oder automatisierten Ablauf des erfindungsgemäßen Verfahrens zu ermöglichen.

Dies kann vorteilhaft dazu beitragen, den zur Unterbrechung der Blutbehandlung erforderlichen Aufwand zu verringern.

Die Steuer- oder Regeleinrichtung kann die Ausführung aller oder im Wesentlichen aller Verfahrensschritte veranlassen. Das erfindungsgemäße Verfahren kann im Wesentlichen oder vollständig von der Steuer- oder Regeleinrichtung ausgeführt werden. Es kann teilweise oder vollständig von der Steuer- oder Regeleinrichtung ausgeführt werden.

In einer bevorzugten Ausführungsform wird die Steuer- oder Regeleinrichtung angesteuert, um zum Zweck der vorübergehenden Unterbrechung der Blutbehandlung eine Blutrückgabe des im extrakorporalen Blutschlauchsatz (Blutschlauchsatz, Blutkassette, Disposable) befindlichen Bluts an den Patienten zu veranlassen.

Alternativ oder ergänzend kann die Steuer- oder Regeleinrichtung die Blutbehandlungssitzung ohne Blutrückgabe an den Patienten unterbrechen.

In einer bevorzugten Ausführungsform wird die Steuer- oder Regeleinrichtung durch Betätigen nur einer Betätigungseinrichtung angesteuert, um die Blutbehandlungsvorrichtung in den Zustand zu versetzen, in welchem die Blutbehandlungssitzung des Patienten zu deren Wiederaufnahme vorübergehend unterbrochen werden kann oder ist, oder in welchem Zustand der Patient ohne weitere Maßnahmen - insbesondere an der Blutbehandlungsvorrichtung - dekonnektierbar ist.

In einer bevorzugten Ausführungsform wird die Steuer- oder Regeleinrichtung durch nur einmaliges Betätigen - wie z. B. einen Tastendruck, ein Bewegen eines Reglers oder dergleichen - nur einer Betätigungseinrichtung angesteuert, um die Blutbehandlungsvorrichtung in den Zustand zu versetzen, in welchem die Blutbehandlungssitzung des Patienten zu deren Wiederaufnahme vorübergehend unterbrochen werden kann.

Die Betätigung oder Ansteuerung der Steuer- oder Regeleinrichtung kann durch Drücken, Drehen, Schalten, usw. der Betätigungseinrichtung erfolgen.

Geeignete Betätigungseinrichtungen schließen einen Knopf, eine Taste, einen Softkey, einen Hardkey, einen Schalter, einen Regler, einen Button eines Touchpads, einen Button eines Touchscreens, einen mittels einer externen Eingabeeinrichtung wie einer Tastatur, einer Maus, einem Stift und dergleichen, bedien- bzw. betätigbaren Button, eine Spracheingabeeinrichtung oder dergleichen ein.

Die Steuer- oder Regeleinrichtung kann von einem Anwender wie Klinikpersonal z.B. Ärzten, Pflegepersonal, dem Patienten (z. B. bei Heimdialyse) und dergleichen bedient oder betätigt werden.

Die Steuer- oder Regeleinrichtung kann von einer weiteren Einrichtung mittels Signalübertragung betätigt werden.

Durch Betätigung oder Ansteuerung der Steuer- oder Regeleinrichtung kann automatisch ein Prozess im Hintergrund gestartet werden, der den Anwender in den verschiedenen Phasen der Blutbehandlungsunterbrechung unterstützt.

Die einzelnen Schritte der Blutbehandlungsunterbrechung können mit Meldungen flankiert werden.

Diese Meldungen können eine Bestätigung durch den Anmelder benötigen.

Die Meldungen können den Anwender über die nächsten Arbeitsschritte informieren.

Auf diese Weise kann es vorteilhaft möglich sein, eine besondere Kontrolle des erfindungsgemäßen Verfahrens durch den Anwender zu ermöglichen.

Das Verfahren kann das Starten der Blutrückgabe umfassen, indem die Steuer- oder Regeleinrichtung wenigstens einen der folgenden Prozesse veranlasst: Dekonnektieren der Blutentnahmeleitung des extrakorporalen Blutkreislaufs vom Blutkreislauf des Patienten; Starten der Blutpumpe und/oder Öffnen der Patientenschlauchklemmen der Blutentnahmeleitung und der Blutrückgabeleitung des extrakorporalen Blutkreislaufs.

Der Begriff "Veranlassen", wie er hierin verwendet wird, meint, dass die Steuer- oder Regeleinrichtung dazu vorgesehen und konfiguriert ist, einen Prozess selbst durchzuführen.

Die Steuer- oder Regeleinrichtung kann dazu vorgesehen oder konfiguriert sein, um - z. B. mittels visueller und/oder akustischer Meldung - anzuzeigen, dass ein Prozess durchgeführt werden muss. Beispielsweise kann die Steuer- oder Regeleinrichtung einen Anwender zur Durchführung eines manuellen Prozesses auffordern.

Der Begriff "Blutrückgabe", wie er hierin verwendet wird, bezeichnet einen Verfahrensschritt oder Prozess, bei welchem extrakorporal behandeltes Blut (alles oder Teile hiervon) des Patienten, welches sich beispielsweise in einem extrakorporalen Blutkreislauf befindet, in den Blutkreislauf des Patienten reinfundiert wird.

Das bei einer Blutrückgabe in den Blutkreislauf des Patienten reinfundierte Blutvolumen kann dabei beispielsweise zwischen 0 und 300 ml betragen. Ein Reinfusionsvorgang kann solange fortgesetzt oder wiederholt werden, bis eine im extrakorporalen Blutkreislauf vorhandene Erfassungseinrichtung, wie ein optischer Detektor, eine optische Dichte "OD hell" erfasst bzw. feststellt, deren Wert beispielsweise vorgegebenen sein kann, die auf die Abwesenheit von Blut und/oder im Wesentlichen ausschließliche Anwesenheit von Substituatflüssigkeit schließen lässt. Beispielsweise kann die Reinfusion von - einmalig oder zunächst - 30 ml fortgesetzt werden. Dies kann dem restlichen Blutvolumen im venösen Leitungsabschnitt entsprechen. Weiteres Blut kann reinfundiert werden. Die Reinfusion kann anschließend beendet werden.

Ein geeignetes Verfahren zur Blutrückgabe kann der Anmeldung der vorliegenden Anmelderin mit dem Titel *"Verfahren zum Entfernen von Blut aus einem extrakorporalen Blutkreislauf für eine Blutbehandlungsvorrichtung nach Beenden einer Blutbehandlungssitzung und Vorrichtung zum Ausführen desselben",* welche am 11. Februar 2009 beim Deutschen Patent- und Markenamt hinterlegt wurde und die Anmeldenummer 10 2009 008 346.4 (Anwalts-Aktenzeichen: FM19A20 DE) trägt, entnommen werden, auf deren diesbezügliche Offenbarung hiermit vollinhaltlich Bezug genommen wird.

Der Begriff "Dekonnektieren" (oder auch Diskonnektieren), wie er hierin verwendet wird, bezeichnet ein Lösen einer Verbindung oder Anbindung des Patienten an einen extrakorporalen Blutkreislauf. Dies kann beispielsweise das Trennen einer Verbindung bzw. eines Zugangs, wie eines Katheters, eines Shunts, einer Fistel, einer Kanüle, einer Nadel (z.B. bei veno-venöser Konnektion) und dergleichen, zwischen dem Blutkreislauf des Patienten und dem extrakorporalen Blutkreislauf einschließen.

Ein "Dekonnektieren der Blutentnahmeleitung des extrakorporalen Blutkreislaufs vom Blutkreislauf des Patienten" kann dabei das Schließen einer Patientenschlauchklemme der Blutentnahmeleitung und/oder das Lösen der Blutentnahmeleitung des extrakorporalen Blutkreislaufs vom Blutkreislauf des Patienten beinhalten.

Zum Beispiel kann das "arterielle Dekonnektieren" des Patienten das Schließen einer arteriellen Patientenschlauchklemme, welche in einem arteriellen Leitungsabschnitt des extrakorporalen Blutkreislaufs angeordnet ist und/oder das Lösen des arteriellen Leitungsabschnitts des extrakorporalen Blutkreislaufs vom arteriellen Zweig eines Patientenzugangs beinhalten.

Die Blutentnahmeleitung des extrakorporalen Blutkreislaufs bzw. Schlauchsystems kann an einen Kochsalzbeutel angeschlossen werden.

Eine Blutpumpe kann während der Blutrückgabe mit einer Geschwindigkeit fördern, bei welcher ein Blutfluss kleiner oder gleich 100 ml/min beträgt.

Nachdem der Patient von der Blutentnahmeleitung dekonnektiert und die Patientenschlauchklemmen der Blutentnahmeleitung und der Blutrückgabeleitung geöffnet wurden, kann die Blutrückgabe des sich im extrakorporalen Blutkreislauf befindlichen Bluts über die Blutrückgabeleitung, z. B. je nach Blutbehandlungsverfahren eine arterielle oder eine venöse Konnektionsnadel, in den Patienten bzw. in den Blutkreislauf des Patienten erfolgen.

In einer weiteren bevorzugten Ausführungsform des erfindungsgemäßen Verfahrens umfasst das Verfahren das Einleiten der Unterbrechung der Blutbehandlung, indem die Steuer- oder Regeleinrichtung wenigstens einen der folgenden Prozesse veranlasst: Stoppen einer Blutpumpe, Schließen einer Patientenschlauchklemme einer Blutentnahmeleitung und einer Patientenschlauchklemme einer Blutrückgabeleitung eines extrakorporalen Blutkreislaufs, Abbrechen einer Heparingabe (als Bolus oder als kontinuierliche Gabe), Beenden einer (kontinuierlichen) Antikoagulationsgabe, insbesondere Citrat, Beenden einer Bilanzierung und/oder Beenden einer Substituatgabe.

Die Steuer- oder Regeleinrichtung kann einen, mehrere oder alle der vorstehend angegebenen Prozesse veranlassen.

Dazu kann die Steuer- oder Regeleinrichtung dazu ausgelegt und vorgesehen sein, um entsprechende Fördereinrichtungen, Ventileinrichtungen, Durchflussregeleinrichtungen und dergleichen anzusteuern und/oder einen Anwender darauf aufmerksam zu machen, einen Prozessschritt auszuführen oder einzuleiten.

Die Steuer- oder Regeleinrichtung kann dazu ausgelegt und vorgesehen oder konfiguriert sein, um alle vorstehend genannten Prozesse gleichzeitig zu veranlassen.

Der Begriff "Bilanzierung", wie er hierin verwendet wird, betrifft die dem extrakorporalen Blut zugeführten Komponenten und diesem entnommene Komponenten.

Beispiele schließen Infusionen, Albumine, Blutkonzentrate, Blutersatzkonzentrate wie die Gabe von Substituat, Medikamentenlösungen, Medikamenten wie Antikoagulationsmitteln wie Heparin, die Gabe von Citrat, Calcium und dergleichen sowie jede Art von Flüssigkeitsvolumina ein.

Der Begriff "Substituatgabe", wie er hierin verwendet wird, bezeichnet die Verabreichung bzw. Zuführung wenigstens einer Substituatflüssigkeit in den extrakorporalen Blutkreislauf des Patienten. Geeignete Substituatflüssigkeiten zur Infusion schließen z.B. isotone Kochsalzlösungen, wie 0,9%-ige NaCl-Lösung, ein.

Das erfindungsgemäße Verfahren kann das Konnektieren einer Blutentnahmeleitung des extrakorporalen Blutkreislaufs mit einer Substituatquelle umfassen.

In einer weiteren bevorzugten Ausführungsform umfasst das Verfahren das Dekonnektieren der Blutrückgabeleitung des extrakorporalen Blutkreislaufs vom Blutkreislauf des Patienten, das Konnektieren der Blutrückgabeleitung des extrakorporalen Blutkreislaufs mit der Substituatquelle und das Zirkulieren wenigstens einer Substituatflüssigkeit durch wenigstens einen Abschnitt des extrakorporalen Blutkreislaufs.

Nach dem Dekonnektieren der Blutrückgabeleitung kann der Patient nunmehr im Wesentlichen oder vollständig vom extrakorporalen Blutkreislauf separiert sein.

Das vorübergehende Unterbrechen einer Blutbehandlungssitzung mit zusätzlichem Dekonnektieren eines Patienten kann vorteilhaft beispielsweise während einer ECCT-Therapie (extracorporeal critical care therapy) (CRRT, continuous renal replacement therapy = Kontinuierliche Nierenersatztherapie, TA = Therapeutische Apherese, Leberersatztherapie) in verschiedenen Situationen zum Einsatz kommen.

Das vorübergehende Unterbrechen erlaubt, dass der Patient kurzzeitig für weitergehende Untersuchungen und/oder Therapien in eine andere Abteilung gesendet werden kann, es erlaubt das Überprüfen der Katheterlage, das Legen eines neuen Katheters, das Vorhandensein eines Zugangsproblems, wie eines Zugangsproblems zum Blutkreislauf eines Patienten, das Aufsuchen der Toilette oder dergleichen.

Das erfindungsgemäße Verfahren zum vorübergehenden Unterbrechen der extrakorporalen Blutbehandlung kann vorteilhaft dazu beitragen, eine Blutbehandlungssitzung zu unterbrechen, ohne die Blutbehandlung komplett beenden zu müssen und damit beispielsweise das Schlauchsystem des extrakorporalen Blutkreislaufs vollständig verwerfen zu müssen.

Wenn der Patient vom extrakorporalen Blutkreislauf dekonnektiert wurde, kann die Zirkulation von Substituatflüssigkeit vorteilhaft dazu dienen, den extrakorporalen Blutkreislauf für eine Wiederaufnahme der Blutbehandlungssitzung vorbereitet zu halten.

Dabei können zur Zirkulation von Substituatflüssigkeit durch den extrakorporalen Blutkreislauf Bedingungen eingestellt bzw. vorgegeben werden, die sich von den Bedingungen und/oder eingestellten Parametern der Blutbehandlungssitzung unterscheiden. Beispielsweise können eine Förderleistung und/oder eine Fördergeschwindigkeit der Blutpumpe reduziert sein.

Es kann möglich sein, einem Anwender Informationen zum laufenden Prozessfortschritt und/oder zur Dauer der laufenden Blutbehandlungsunterbrechung zu geben. Beispielsweise kann der Anwender eine Meldung auf einem Bildschirm oder dergleichen erhalten, welche ihm die Möglichkeit bieten kann, die laufende Blutbehandlungsunterbrechung jederzeit abzubrechen. Das Abbrechen der Zirkulation kann mittels einer geeigneten Eingabeeinrichtung, wie beispielsweise eines Stop-Hardkeys, und/oder über ein Informationsfenster eingeleitet werden.

Auch kann es möglich sein, Parameter sowie Grenzwertfenster dieses Parameters, wie beispielsweise des Drucks, im Vergleich zur Blutbehandlungssitzung zu verändern. Grenzwertfenster können deaktiviert werden. Es können Skalenenden, d. h. beispielsweise ein oberer Grenzwert oder Grenzwertbereich und/oder ein unterer Grenzwert oder Grenzwertbereich, deaktiviert werden. Beispielsweise kann ein unteres venöses Skalenende deaktiviert werden. Ebenso oder zusätzlich dazu kann es möglich sein, ein oberes arterielles Skalenende zu deaktivieren. Eine Überwachung von behandlungsrelevanten Parametern kann durch diese Maßnahme entsprechend während der Zirkulation abgeschaltet werden. Auf diese Weise kann es vorteilhaft möglich sein, mögliche falschpositive Alarme während der Zirkulation zu vermeiden.

Die Zirkulation kann abgebrochen werden, wenn z. B. eine Erfassungseinrichtung zum Erfassen einer Zusammensetzung des Leitungsinhalts des extrakorporalen Blutkreislaufs, wie beispielsweise ein optischer Detektor, eine bestimmte, z. B. vorgegebene oder voreingestellte, optische Dichte "OD dunkel" erkennt. Es kann ein Rücksprung in die Blutbehandlung bzw. eine Wiederaufnahme der Blutbehandlungssitzung erfolgen.

Wenn beispielsweise eine zunächst als maximal bestimmte Zirkulationsdauer - hier exemplarisch mit z. B. 4h angegeben - überschritten wird (oder rechtzeitig vorher), kann dies zur Ausgabe einer Meldung führen. Die Blutpumpe kann gestoppt werden. Anschließend kann es möglich sein, die Meldung (z. B. durch einen Anwender) zu bestätigen und die Blutbehandlungsunterbrechung bzw. die Zirkulation über die zunächst als maximal bestimmte Zirkulationsdauer - fortzusetzen.

In einer bevorzugten Weiterbildung umfasst das erfindungsgemäße Verfahren das Ansteuern der Steuer- oder Regeleinrichtung, um eine Wiederaufnahme der Blutbehandlungssitzung bei dem erneut mit der Blutbehandlungsvorrichtung konnektierten Patienten zu veranlassen.

Das Konnektieren des Patienten kann, muss aber nicht, Teil des erfindungsgemäßen Verfahrens sein.

Der Begriff "Wiederaufnahme der Blutbehandlungssitzung", wie er hierin verwendet wird, bezeichnet eine Fortsetzung der Blutbehandlungssitzung.

Die Blutbehandlungssitzung kann mit den gleichen Parametern und/oder unter den gleichen Bedingungen fortgesetzt werden, die zu Beginn der Blutbehandlungssitzung und/oder während des Ablaufs der Blutbehandlungssitzung vor ihrer Unterbrechung eingestellt worden waren.

Die Steuer- oder Regeleinrichtung kann durch Betätigen wie Drücken, Drehen, Schalten, usw. nur einer, ggf. auch mehrerer, Betätigungseinrichtung(en) angesteuert werden, um eine Wiederaufnahme der Blutbehandlungssitzung des erneut mit der Blutbehandlungsvorrichtung konnektierten Patienten nach deren vorübergehender Unterbrechung zu veranlassen.

Zur Fortsetzung der Blutbehandlungssitzung kann ein arterielles und venöses Konnektieren des Patienten, z. B. also das Konnektieren der Blutentnahmeleitung und der Blutrückgabeleitung des extrakorporalen Blutkreislaufs, mit dem Blutkreislauf des Patienten, ein Starten der Blutpumpe und ein Fördern eines Leitungsinhalts im Inneren des extrakorporalen Blutkreislaufs, bis eine optische Dichte (OD) des Leitungsinhalts eine vorgegebene "OD dunkel" erreicht hat, ein Stoppen der Blutpumpe; und ein Schließen der Patientenschlauchklemmen der Blutentnahmeleitung und der Blutrückgabeleitung ausgeführt werden.

Die vorstehend genannten Prozesse können von der Steuer- oder Regeleinrichtung initiiert oder ausgelöst werden.

Die Aufgabe der vorliegenden Erfindung wird ferner durch eine Steuer- oder Regeleinrichtung gemäß Anspruch 9 gelöst. Alle mit dem erfindungsgemäßen Verfahren erzielbaren Vorteile lassen sich ungeschmälert auch mit der erfindungsgemäßen Steuer- oder Regeleinrichtung erreichen.

Die Steuer- oder Regeleinrichtung der vorliegenden Erfindung ist dazu vorgesehen und konfiguriert, angesteuert zu werden, um die Blutbehandlung mittels einer Blutbehandlungsvorrichtung vorübergehend zu unterbrechen.

Die Unterbrechung der Blutbehandlungssitzung kann mit dem Ziel der Wiederaufnahme der extrakorporalen Blutbehandlung erfolgen.

Die erfindungsgemäße Steuer- oder Regeleinrichtung kann dazu vorgesehen und konfiguriert sein, zum vorübergehenden Unterbrechen der Blutbehandlungsvorrichtung in einem Verfahren der vorliegenden Erfindung verwendet zu werden.

Die Steuer- oder Regeleinrichtung kann dazu vorgesehen und konfiguriert sein, durch Betätigen nur einer Betätigungseinrichtung des Anwenders angesteuert zu werden.

Die Steuer- oder Regeleinrichtung kann dazu vorgesehen und konfiguriert sein, eine Wiederaufnahme der Blutbehandlungssitzung zu veranlassen.

Die Steuer- oder Regeleinrichtung kann beispielsweise dazu vorgesehen und konfiguriert sein, um durch Betätigen nur einer Betätigungseinrichtung des Anwenders eine Wiederaufnahme der Blutbehandlungssitzung des Patienten nach deren vorübergehender Unterbrechung zu veranlassen.

Die Aufgabe der vorliegenden Erfindung wird ferner durch eine Blutbehandlungsvorrichtung gemäß Anspruch 12 gelöst. Alle mit dem erfindungsgemäßen Verfahren erzielbaren Vorteile lassen sich ungeschmälert wiederum auch mit der erfindungsgemäßen Blutbehandlungsvorrichtung erreichen.

Die erfindungsgemäße Blutbehandlungsvorrichtung ist zur extrakorporalen Blutbehandlung wie beispielsweise einer Dialyse, einer Hämofiltration, einer Hämodiafiltration, einer Blutbegasung, einer Apherese, einer Adsorption und dergleichen geeignet und/oder vorgesehen bzw. konfiguriert.

Die Blutbehandlungsvorrichtung weist wenigstens eine Steuer- oder Regeleinrichtung gemäß der vorliegenden Erfindung auf.

Die Blutbehandlungsvorrichtung kann ferner weitere Einrichtungen wie Ein- und/oder Ausgabeeinrichtungen, Speichereinrichtungen und dergleichen aufweisen und/oder mit solchen Einrichtungen koppelbar sein.

Die Blutbehandlungsvorrichtung kann insbesondere wenigstens eine Betätigungseinrichtung aufweisen. Die Betätigungseinrichtung kann dazu vorgesehen und ausgestaltet sein, um ein Ansteuern der Steuer- oder Regeleinrichtung zu ermöglichen. Ein solches Ansteuern kann beispielsweise durch einmaliges Betätigen der Betätigungseinrichtung erreicht bzw. ausgelöst werden.

Die Aufgabe der vorliegenden Erfindung wird ferner durch ein digitales Speichermedium gemäß Anspruch 14 und/oder ein Computer-Programm-Produkt gemäß Anspruch 15 und/oder ein Computer-Programm gemäß Anspruch 16 gelöst. Alle mit dem erfindungsgemäßen Verfahren erzielbaren Vorteile lassen sich ungeschmälert auch mit dem erfindungsgemäßen digitalen Speichermedium, dem erfindungsgemäßen Computer-Programm-Produkt und/oder dem erfindungsgemäßen Computer-Programm erreichen.

Das digitale Speichermedium, welches insbesondere eine Diskette, eine CD oder eine DVD ist, weist bevorzugt elektrisch auslesbare Steuersignale auf, die so mit einem programmierbaren Computersystem zusammenwirken können, dass die maschinellen Schritte des erfindungsgemäßen Verfahrens veranlasst werden.

Dabei können alle, einige oder manche der maschinell durchgeführten Schritte des erfindungsgemäßen Verfahrens veranlasst werden. Letzteres gilt auch für das Computer-Programm-Produkt und das Computer-Programm.

Das Computer-Programm-Produkt weist vorzugsweise einen auf einem maschinenlesbaren Träger gespeichertem Programmcode zur Veranlassung der maschinell durchführbaren Schritte des erfindungsgemäßen Verfahrens, wenn das Programm-Produkt auf einem Rechner abläuft, auf.

Der Begriff "maschinenlesbarer Träger", wie er hierin verwendet wird, bezeichnet einen Träger, der von Software und/oder Hardware interpretierbare Daten oder Informationen enthält. Der Träger kann ein Datenträger, wie eine Diskette, eine CD, DVD und dergleichen sein.

Das Computer-Programm weist einen Programmcode zur Veranlassung der maschinell durchführbaren Schritte des erfindungsgemäßen Verfahrens auf, wenn das Programm auf einem Rechner oder Computer abläuft.

Die vorliegende Erfindung kann vorteilhaft eine vereinfachte Unterbrechung einer Blutbehandlungssitzung ermöglichen.

Mittels der Steuer- oder Regeleinrichtung kann es dabei vorteilhaft möglich sein, mehrere Aktionen gleichzeitig auszulösen bzw. zu veranlassen und/oder durchzuführen. Die vorliegende Erfindung kann somit vorteilhaft eine Vereinfachung der Unterbrechung einer Blutbehandlungssitzung bereitstellen. Ferner kann mittels der vorliegenden Erfindung Zeit dadurch eingespart werden, dass mehrere Schritte praktisch gleichzeitig veranlasst werden können. Zudem können die Schritte, welche aufeinander folgen, aufgrund ihres Auslösens durch die erfindungsgemäße Steuer- oder Regeleinrichtung optimal aufeinander abgestimmt werden.

Das ledigliche Unterbrechen der Blutbehandlungssitzung im Bedarfsfall kann gegenüber einer Beendigung bzw. einem vollständigen Abbruch der Blutbehandlungssitzung von Vorteil sein. Zum einen kann es vorteilhaft dazu beitragen, Kosten für den Kunden und/oder Patienten zu senken. Zum Beispiel kann das Schlauchsystem für CRRT-Blutbehandlungen bis zu 72 h eingesetzt werden und kann vorteilhaft auch bei einer kurzzeitigen Blutbehandlungsunterbrechung im Anschluss wieder bis zum Erreichen einer max. Blutbehandlungszeit von 72 h zur Verfügung stehen.

Eine Tagesplanung der Untersuchungen des Patienten und/oder weiterführende Blutbehandlungen können vorteilhaft unabhängig von der Blutbehandlungssitzung durchgeführt werden.

Die vorliegende Erfindung kann vorteilhaft eine hohe Anwenderfreundlichkeit bereitstellen. Mögliche manuelle Arbeitsschritte können weitestgehend verringert oder sogar vollständig eingestellt werden. Zudem kann es vorteilhaft möglich sein, ein simultanes Verändern von Parametern, welche an der Behandlungsvorrichtung oder im extrakorporalen Blutkreislauf herrschen, aufeinander abgestimmt durchzuführen. Vorteilhafterweise kann die Anpassung von Parametern, wie beispielsweise eine Reduzierung des Blutflusses und dergleichen, ebenfalls automatisiert und in Abhängigkeit von anderen, aus der Vorbereitung zur Unterbrechung sich ergebenden Parametern erfolgen.

Da die Steuer- oder Regeleinrichtung mit Einrichtungen zum Anzeigen und/oder Ausgeben von Meldungen und dergleichen ausgestattet oder verbunden sein kann, kann ein Anwender vorteilhaft über alle oder im Wesentlichen alle Arbeitsschritte informiert und/oder entsprechend durch den aktuell ablaufenden Prozess(-schritt) geführt werden. Rückkoppelungen zwischen Sensoren, welche Ergebnisse von Messungen der beim Unterbrechen betroffenen Parameter melden, sind dabei möglich und erfindungsgemäß in bestimmten Ausführungsformen vorgesehen.

Die vorliegende Erfindung kann vorteilhaft eine erhöhte Sicherheit des Gesamtsystems bereitstellen. So kann es beispielsweise vorteilhaft möglich sein, das Reinfusionsvolumen während der laufenden Reinfusion zu überwachen und/oder anzuzeigen. Es kann ein maximales Reinfusionsvolumen überwacht werden. Wenn das maximale Reinfusionsvolumen überschritten werden sollte, kann entsprechend vorteilhaft eine Meldung ausgegeben und der Reinfusionsvorgang möglicherweise vollständig gestoppt werden.

Ferner kann es durch Überwachung der maximalen Dauer einer Blutbehandlungsunterbrechung möglich sein, eine erhöhte Sicherheit des Gesamtsystems bereitzustellen.

Im Folgenden wird die vorliegende Erfindung beispielhaft unter Bezugnahme auf die beigefügte Zeichnung beschrieben. Es gilt:
- Fig. 1: zeigt eine schematische Übersicht einer beispielhaften Anordnung zur Durchführung des erfindungsgemäßen Verfahrens; und
- Fig. 2: zeigt einen Ablaufplan des erfindungsgemäßen Verfahrens.

Wie vorstehend angegeben, gibt es extrakorporale Blutbehandlungsverfahren, bei denen die Blutentnahmeleitung eines extrakorporalen Blutkreislaufs der arterielle Leitungsabschnitt des extrakorporalen Blutkreislaufs ist, und bei welchen die Blutrückgabeleitung entsprechend der venöse Leitungsabschnitt ist. Beispiele hierfür schließen Blutbehandlungsverfahren wie extrakorporale Blutreinigungsverfahren, z. B. Hämodialyse, Hämofiltration, Hämodiafiltration, und dergleichen ein.

In anderen extrakorporalen Blutbehandlungsverfahren, z. B. Blutbegasungsverfahren mit Sauerstoff, kann die Entnahmerichtung gegenüber derjenigen einer z. B. Hämodialyse derart umgekehrt sein, dass Blut über die Blutentnahmeleitung aus dem venösen Schenkel des Gefäßsystems des Patienten entnommen und nach seiner Anreicherung mit Sauerstoff über die Blutrückgabeleitung in den arteriellen Schenkel des Gefäßsystems des Patienten zurückgegeben wird.

In den folgenden Ausführungsformen wird die Erfindung beispielhaft anhand einer arteriellen Blutentnahmeleitung und einer venösen Blutrückgabeleitung beschreiben. Wie ein Fachmann jedoch leicht erkennen wird, haben die Entnahme- und Rückgaberichtung oder -stelle des Bluts keinen Einfluss auf die Durchführbarkeit des erfindungsgemäßen Verfahrens. Das Verfahren soll nicht auf diese Ausführungsform beschränkt werden.

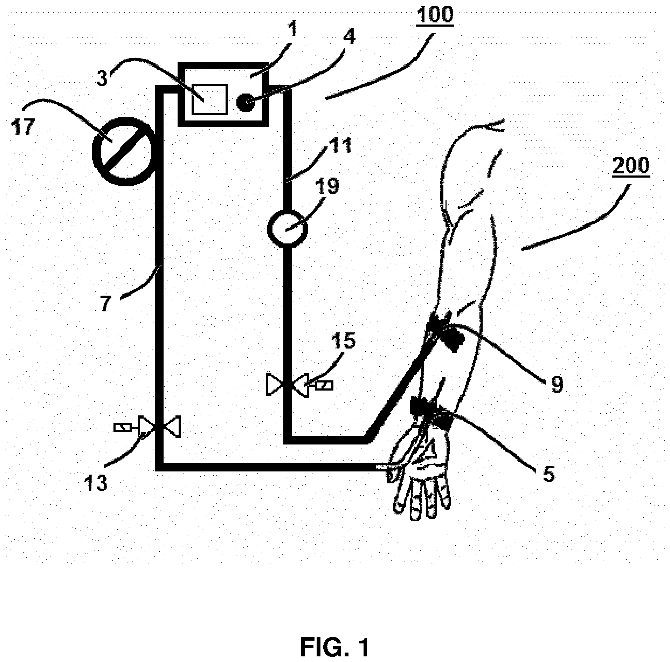

**Fig. 1** zeigt eine schematische Übersicht einer Anordnung zur Durchführung einer Blutbehandlungssitzung.

Fig. 1 zeigt einen extrakorporalen Blutkreislauf 100, verbunden mit einer erfindungsgemäßen Blutbehandlungsvorrichtung 1. Die Blutbehandlungsvorrichtung 1 weist eine Steuer- oder Regeleinrichtung 3 und eine Betätigungseinrichtung 4 auf.

Ein Patient 200 ist über einen Patientenzugang mit dem extrakorporalen Blutkreislauf 100 verbunden. Genauer gesagt, ist der Patient 200 über einen ersten Patientenzugang, hier einen arteriellen Patientenzugang 5, mit einer Blutentnahmeleitung, hier einem arteriellen Leitungsabschnitt 7, des extrakorporalen Blutkreislaufs 100 verbunden. Der Patient 200 ist über einen zweiten Patientenzugang, hier einen venösen Patientenzugang 9, mit einer Blutrückgabeleitung, hier einem venösen Leitungsabschnitt 11, des extrakorporalen Blutkreislaufs 100 verbunden.

Im arteriellen Leitungsabschnitt 7 ist eine erste Patientenschlauchklemme, hier eine arterielle Patientenschlauchklemme 13, angeordnet. Im venösen Leitungsabschnitt 11 ist eine zweite Patientenschlauchklemme, hier eine venöse Patientenschlauchklemme 15, angeordnet.

In Fig. 1 sind die arterielle Patientenschlauchklemme 13 und die venöse Patientenschlauchklemme 15 in einem geöffneten Zustand gezeigt.

Blut des Patienten 200 wird mittels einer Blutpumpe 17 innerhalb des extrakorporalen Blutkreislaufs 100 gefördert.

Im venösen Leitungsabschnitt 11 ist eine Erfassungseinrichtung, z. B. ein optischer Detektor 19, vorgesehen, welcher dazu ausgelegt und vorgesehen sein kann, eine optische Dichte (OD) eines im Inneren des extrakorporalen Blutkreislaufs 100 vorhandenen Leitungsinhalts zu erfassen.

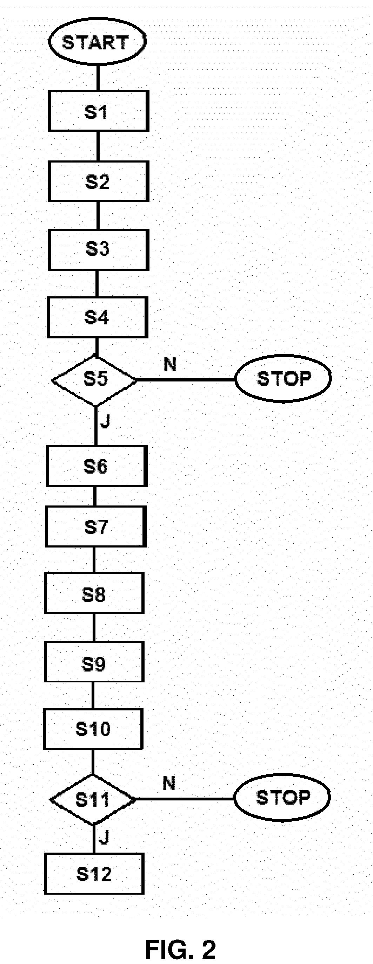

**Fig. 2** zeigt schematisch einen Ablaufplan eines erfindungsgemäßen Verfahrens zum vorübergehenden Unterbrechen einer extrakorporalen Blutbehandlung eines Patienten.

Zum leichteren Verständnis des Zusammenspiels der einzelnen Komponenten wird auf die in Fig. 1 gezeigte Übersicht Bezug genommen. Die im erfindungsgemäßen Verfahren auftretenden Komponenten sind daher im Folgenden mit den entsprechenden in Fig. 1 gezeigten Bezugszeichen genannt, obwohl sie ggf. nicht auch in Fig. 2 dargestellt sind.

Zunächst kann ein Anwender in einem Menü einen Softkey "Blutbehandlungsunterbrechung" auswählen (in Fig. 2 nicht gezeigt).

Im vorliegenden Fall wählt der Anwender in einem Schritt S1 die Blutbehandlungsunterbrechung mit Blutrückgabe aus. Die Auswahl kann mittels - vorzugsweise nur einer Betätigung - einer Betätigungseinrichtung 4 wie eines Softkeys erfolgen. Der Softkey kann auf einem (nicht gezeigten) Bildschirm der Blutbehandlungsvorrichtung 1 angeordnet sein. Die Betätigung kann allerdings auch auf jede andere, dem Fachmann geläufige Weise erfolgen.

In dem sich anschließenden Schritt S2 veranlasst die Steuer- oder Regeleinrichtung 3 den Stopp der Blutpumpe 17 des extrakorporalen Blutkreislaufs 100.

Dabei können eine arterielle Patientenschlauchklemme 13 und/oder eine venöse Patientenschlauchklemme 15 geschlossen werden.

Die Steuer- oder Regeleinrichtung 3 kann ferner das Abbrechen eines Heparinbolus, das Stoppen einer kontinuierlichen Antikoagulation wie Heparin, Citrat, Ci-Ca (Citrat-Calcium) mit Ca als Antagonist der Antikoagulation, das Stoppen einer Bilanzierung und/oder das Abbrechen eines Substituat-Bolus veranlassen.

Auf dem Bildschirm der Blutbehandlungsvorrichtung 1 können Anweisungen zum weiteren Vorgehen und/oder Anzeigen zum laufenden Prozessschritt angezeigt werden.

Beispielsweise kann einem Anwender mitgeteilt werden, dass der Patient 200 arteriell zu dekonnektieren ist. Der Anwender kann in einem Schritt S3 dazu aufgefordert werden, eine Dekonnektion des Patienten 200 vorzunehmen.

Der arterielle Leitungsabschnitt 7 des Schlauchsystems des extrakorporalen Blutkreislaufs 100 wird sodann vorzugsweise an eine Substituatquelle, wie einen Kochsalzbeutel, angeschlossen.

Vor Starten einer Reinfusion können alle Konnektionen auf ihre Dichtigkeit überprüft werden.

Die kontinuierliche Antikoagulation kann weiterhin ausgesetzt bleiben. Die Bilanzierung kann weiterhin ausgesetzt bleiben.

Die Reinfusion kann in einem Schritt S4 gestartet werden, indem die Steuer- oder Regeleinrichtung 3 - vorzugsweise durch Betätigung wiederum eines anderen Softkeys - die Reinfusion veranlasst.

Zum Durchführen der Reinfusion können die arterielle Patientenschlauchklemme 13 und die venöse Patientenschlauchklemme 15 geöffnet werden. Die Blutpumpe 17 kann gestartet werden. Die Blutpumpe 17 kann beispielsweise mit einer Fördergeschwindigkeit von ≤ 100 ml/min betrieben werden.

Ein Blutfluss von 100 ml/min kann beispielsweise bedeuten, dass der Blutfluss während der Reinfusion - im Vergleich zum Blutfluss während der Blutbehandlungssitzung - zu reduzieren ist. Falls der Blutfluss bereits während der Blutbehandlungssitzung < 100 ml/min betragen hat, kann die Fördergeschwindigkeit der Blutpumpe 17 (auf diesem niedrigeren Wert als 100 ml) beibehalten werden. Aber auch Geschwindigkeiten über 100 ml/min sind erfindungsgemäß für die Reinfusion möglich.

Auf dem Bildschirm der Blutbehandlungsvorrichtung 1 können dem Anwender wiederum Anweisungen zum weiteren Vorgehen und/oder Anzeigen zum laufenden Prozessschritt gegeben werden. Beispielsweise kann dem Anwender eine Information zum laufenden Prozessschritt und/oder zu dem bereits verabreichten Reinfusionsvolumen gegeben werden.

Im Hintergrund können weitere Prozesse und/oder Überwachungen stattfinden. Diese können sich von den während einer Blutbehandlungssitzung ablaufenden Prozessen unterscheiden.

Im Schritt S5 kann abgefragt werden, ob eine Erfassungseinrichtung, wie ein optischer Detektor 19, erkennt, ob eine optische Dichte mit dem Zielwert "OD hell" innerhalb eines vorgegebenen Volumens vorliegt.

Das Vorliegen des Werts für "OD hell" kann anzeigen, dass sich im Wesentlichen kein Blut mehr in einem Leitungsinhalt im Inneren des extrakorporalen Blutkreislaufs 100 befindet. Der Wert "OD hell" kann vorgegeben sein. Er kann z. B. intern in einer Speichereinrichtung und/oder in einer Software hinterlegt sein.

Wird der Wert "OD hell" nicht erreicht ("N" für "Nein"), kann eine Meldung ausgegeben werden. In jedem Fall kann die Blutpumpe 17 stoppt werden.

Falls der vorgegebene Wert für "OD hell" erreicht wird ("J" für "Ja"), kann ein Stoppen der Blutpumpe 17 durch die Steuer- oder Regeleinrichtung 3 in Schritt S6 veranlasst werden.

Die arterielle Patientenschlauchklemme 13 und die venösen Patientenschlauchklemme 15 werden geschlossen.

Der Patient 200 kann venös dekonnektiert werden. Der Patient 200 ist nun vollständig von der Blutbehandlungsvorrichtung 1 separiert und kann beispielsweise das Blutbehandlungszimmer verlassen.

In Schritt S7 kann sodann mittels der Steuer- oder Regeleinrichtung 3 eine Zirkulation von Substituatflüssigkeit veranlasst werden.

Zunächst kann der Bildschirm der Blutbehandlungsvorrichtung 1 eine Meldung anzeigen, mit welcher abgefragt wird, ob der Patient dekonnektiert wurde.

Wenn der Patient 200 vollständig von der Blutbehandlungsvorrichtung 1 bzw. Teilen derselben, wie dem extrakorporalen Blutkreislauf 100, dekonnektiert wurde, können auf dem Bildschirm Anweisungen zum weiteren Vorgehen und Angaben zum laufenden Prozess angezeigt werden.

Als nächstes ist vorgesehen, den venösen Leitungsabschnitt 11 ebenfalls an die Substituatquelle, z.B. den Kochsalzbeutel, anzuschließen. Dies kann vom Anwender vorgenommen werden.

Alle Konnektionen können auf ihre Dichtigkeit geprüft werden.

Sodann kann die Steuer- oder Regeleinrichtung 3 in Schritt S8 die Zirkulation der Substituatflüssigkeit veranlassen. Dies kann vorteilhaft wiederum über bloßes Betätigen eines Softkeys erreicht werden.

Die kontinuierliche Antikoagulation kann weiterhin ausgesetzt oder angehalten bleiben. Die Bilanzierung kann weiterhin gestoppt bleiben.

Die Blutpumpe 17 kann weiterhin mit einer Fördergeschwindigkeit von ≤ 100 ml/min betrieben werden.

Auf dem Bildschirm können Anweisungen zum weiteren Vorgehen und/oder Anzeigen zum laufenden Prozessschritt und/oder zur Dauer der laufenden Blutbehandlungsunterbrechung ausgegeben werden.

An dieser Stelle kann der Anwender - beispielsweise über eine Meldung oder Anzeige auf dem Bildschirm informiert - die Möglichkeit erhalten, die laufende Blutbehandlungsunterbrechung (jederzeit) abzubrechen.

Im Hintergrund können weitere Prozesse ablaufen und/oder Überwachungen stattfinden. Diese Prozessabläufe können sich von Prozessabläufen während der Blutbehandlungssitzung unterscheiden.

Es können beispielsweise andere Grenzwertfenster, z. B. des Drucks, als während der Blutbehandlung eingestellt werden. Grenzwertfenster können deaktiviert werden. Ein unteres venöses Skalenende kann deaktiviert werden. Ein oberes arterielles Skalenende kann deaktiviert werden.

Die Zirkulation kann beispielsweise abbrechen, wenn eine optische Dichte "OD dunkel" erkannt wird, welche ebenfalls ein in einer Speichereinrichtung hinterlegter Wert sein kann. Es kann ein Rücksprung in die Blutbehandlungssitzung erfolgen.

In einem Fall, in dem eine zunächst als maximal eingestellte Zirkulationsdauer von beispielsweise 4 h überschritten wird, kann eine Ausgabe einer entsprechenden Meldung folgen. Die Blutpumpe 17 kann gestoppt werden. Diese Meldung kann bestätigt und die Blutbehandlungsunterbrechung bzw. Zirkulation kann fortgesetzt werden.

Wenn die Blutbehandlungssitzung wieder aufgenommen werden soll, kann in Schritt S9 durch die Steuer- oder Regeleinrichtung 3 ein Abbruch der Zirkulation von Substituatflüssigkeit veranlasst werden.

Dazu kann zunächst ein Stoppen der Blutpumpe 17, welche während der Zirkulation Substituatflüssigkeit gefördert hat, veranlasst werden.

Die arterielle Patientenschlauchklemme 13 und die venöse Patientenschlauchklemme 15 können geschlossen werden.

Auf dem Bildschirm können wiederum Anweisungen zum weiteren Vorgehen und/oder Anzeigen zum laufenden Prozessschritt erscheinen. Diese können einen Hinweis mit Anweisungen zur Patientenkonnektion umfassen.

Des Weiteren kann die Steuer- oder Regeleinrichtung das Starten der Blutpumpe 17 veranlassen, indem beispielsweise ein Softkey betätigt wird.

Vorzugsweise wird die arterielle Konnektion des Patienten bestätigt. Anschließend kann, z. B. lediglich durch Drücken einer Taste, durch die Steuer- oder Regeleinrichtung 3 veranlasst werden, dass die Blutpumpe 17 gestartet wird. Die Blutpumpe 17 kann weiterhin mit einem Blutfluss von ≤ 100ml/min fördern.

Die arterielle Patientenschlauchklemme 13 und die venöse Patientenschlauchklemme 15 können wieder geöffnet werden.

Auf dem Bildschirm können erneut Anweisungen zum weiteren Vorgehen und/oder Anzeigen zum laufenden Prozessschritt erfolgen.

Daneben können im Hintergrund wiederum verschiedene Prozessabläufe und/oder Überwachungen stattfinden.

Zunächst wird in Schritt S10 begonnen, den Patienten zu konnektieren.

In Schritt S11 findet eine Abfrage statt, ob eine bestimmte (vorgegebene) optische Dichte "OD dunkel" innerhalb eines vorgegebenen Volumens erreicht wurde. Der Wert für die optische Dichte "OD dunkel" kann in einer Speichereinrichtung und/oder in einer Software hinterlegt sein.

Wird der Wert für die "OD dunkel" nicht erreicht, so kann eine entsprechende Meldung ausgegeben und die Blutpumpe 17 gestoppt werden.

Wenn der Wert für die "OD dunkel" erreicht wird, so wird die Blutpumpe 17 gestoppt.

Sodann können die arterielle Patientenschlauchklemme 13 und die venöse Patientenschlauchklemme 15 geschlossen werden. Sobald der Patient 200 (zum Beispiel nach einer anderweitigen Blutbehandlung) wieder verfügbar ist, kann die Prozedur in umgekehrter Reihenfolge durchgeführt werden, so dass die Blutbehandlungssitzung fortgesetzt werden kann.

Um die Blutbehandlung fortzusetzen, kann die Steuer- oder Regeleinrichtung 3 in Schritt S12 veranlassen, die Blutpumpe 17 mit dem Blutfluss erneut zu starten, der in der Blutbehandlung vorlag, bevor die Blutbehandlungsunterbrechung gestartet wurde.

Die arterielle Patientenschlauchklemme 13 und die venöse Patientenschlauchklemme 15 können wieder geöffnet werden.

Die kontinuierliche Antikoagulation (Heparin, Citrat, Ci-Ca, Calcium als Antikoagulant) und die Bilanzierung können wieder gestartet werden.

Alle Überwachungen aus der Blutbehandlungssitzung können wieder aktiviert werden.

## Patentansprüche

1. Verfahren zum vorübergehenden Unterbrechen einer mittels einer Blutbehandlungsvorrichtung (1) durchgeführten extrakorporalen Blutbehandlung eines Patienten (200), wobei der Patient (200) zum Zwecke der Blutbehandlung mit der Blutbehandlungsvorrichtung (1) konnektiert ist, mit dem Schritt:
- Ansteuern einer Steuer- oder Regeleinrichtung (3), welche dazu vorgesehen und konfiguriert ist, um die Blutbehandlungsvorrichtung (1) in einen Zustand zu versetzen, in welchem die Blutbehandlungssitzung durch Dekonnektieren des Patienten (200) von der Blutbehandlungsvorrichtung (1) zu deren Wiederaufnahme vorübergehend unterbrochen werden kann.

2. Verfahren nach Anspruch 1, wobei die Steuer- oder Regeleinrichtung (3) angesteuert wird, um zum Zwecke der vorübergehenden Unterbrechung der Blutbehandlung eine Blutrückgabe von extrakorporal behandeltem Blut an den Patienten (200) zu veranlassen.

3. Verfahren nach einem der vorangegangenen Ansprüche, wobei durch Betätigen nur einer Betätigungseinrichtung (4) die Steuer- oder Regeleinrichtung (3) angesteuert wird, um die Blutbehandlungsvorrichtung (1) in den Zustand zu versetzen, in welchem die Blutbehandlungssitzung des Patienten (200) zu deren Wiederaufnahme vorübergehend unterbrochen werden kann oder ist.

4. Verfahren nach einem der vorangegangenen Ansprüche mit dem Schritt:
- Einleiten der Unterbrechung der Blutbehandlung, indem die Steuer- oder Regeleinrichtung (3) wenigstens einen der folgenden Prozesse veranlasst:
a) Stoppen der Blutpumpe (17);
b) Schließen einer Patientenschlauchklemme einer Blutentnahmeleitung und einer Patientenschlauchklemme einer Blutrückgabeleitung eines extrakorporalen Blutkreislaufs (100);
c) Abbrechen einer Heparingabe;
d) Beenden einer (kontinuierlichen) Antikoagulationsgabe;
e) Beenden einer Bilanzierung;
f) Beenden einer Substituatgabe.

5. Verfahren nach einem der vorangegangenen Ansprüche mit den weiteren Schritten:
- Dekonnektieren einer Blutrückgabeleitung des extrakorporalen Blutkreislaufs (100) vom Blutkreislauf des Patienten (200);
- Konnektieren der Blutrückgabeleitung des extrakorporalen Blutkreislaufs (100) mit der Substituatquelle; und
- Zirkulieren wenigstens einer Substituatflüssigkeit durch wenigstens einen Abschnitt des extrakorporalen Blutkreislaufs (100).

6. Verfahren nach einem der vorangegangenen Ansprüche, mit dem Schritt:
Ansteuern der Steuer- oder Regeleinrichtung (3), um eine Wiederaufnahme der Blutbehandlungssitzung bei erneut mit der Blutbehandlungsvorrichtung (1) konnektiertem Patienten (200) zu veranlassen.

7. Verfahren nach Anspruch 6, wobei durch Betätigen nur einer Betätigungseinrichtung (4) die Steuer- oder Regeleinrichtung (3) angesteuert wird, um eine Wiederaufnahme der Blutbehandlungssitzung des erneut mit der Blutbehandlungsvorrichtung (1) konnektierten Patienten (200) nach deren vorübergehender Unterbrechung zu veranlassen.

8. Verfahren nach Anspruch 6 oder 7 mit den Schritten:
- Konnektieren einer Blutrückgabeleitung und einer Blutentnahmeleitung des extrakorporalen Blutkreislaufs (100) mit dem Blutkreislauf des Patienten (200);
- Starten der Blutpumpe (17) und Fördern eines Leitungsinhalts im Inneren eines extrakorporalen Blutkreislaufs (100), bis eine optische Dichte des Leitungsinhalts einen vorgegebenen Wert erreicht;
- Stoppen der Blutpumpe (17); und
- Schließen der Patientenschlauchklemme der Blutentnahmeleitung und der Patientenschlauchklemme der Blutrückgabeleitung.

9. Steuer- oder Regeleinrichtung (3), welche dazu vorgesehen und konfiguriert ist, angesteuert zu werden, um die Blutbehandlung mittels einer Blutbehandlungsvorrichtung (1) vorübergehend zu unterbrechen, und/oder welche dazu vorgesehen und konfiguriert ist, eine vorübergehende Unterbrechung der Blutbehandlungsvorrichtung (1) in einem Verfahren nach einem der Ansprüche 1 bis 8 zu bewirken.

10. Steuer- oder Regeleinrichtung (3) nach Anspruch 9, welche dazu vorgesehen und konfiguriert ist, durch Betätigen nur einer Betätigungseinrichtung (4) angesteuert zu werden.

11. Steuer- oder Regeleinrichtung (3) nach Anspruch 9 oder 10, welche dazu vorgesehen und konfiguriert ist, um eine Wiederaufnahme der Blutbehandlungssitzung zu veranlassen.

12. Blutbehandlungsvorrichtung (1) zur extrakorporalen Blutbehandlung mit
- wenigstens einer Steuer- oder Regeleinrichtung (3) gemäß einem der Ansprüche 9 bis 11.

13. Blutbehandlungsvorrichtung nach Anspruch 12, ferner aufweisend
- wenigstens eine Betätigungseinrichtung (4), welche dazu vorgesehen und ausgestaltet ist, um ein Ansteuern der Steuer- oder Regeleinrichtung (3) allein durch sie zu ermöglichen.

14. Digitales Speichermedium, insbesondere Diskette, CD oder DVD, mit elektrisch auslesbaren Steuersignalen, die so mit einem programmierbaren Computersystem zusammenwirken können, dass die maschinellen Schritte eines Verfahrens gemäß einem der Ansprüche 1 bis 8 veranlasst werden.

15. Computer-Programm-Produkt mit auf einem maschinenlesbaren Träger gespeichertem Programmcode zur Veranlassung der maschinellen Schritte des Verfahrens gemäß einem der Ansprüche 1 bis 8, wenn das Programm-Produkt auf einem Rechner abläuft.

16. Computer-Programm mit Programmcode zur Veranlassung der maschinellen Schritte des Verfahrens gemäß einem der Ansprüche 1 bis 8, wenn das Programm auf einem Computer abläuft.
